(19) Europäisches Patentamt
European Patent Office
Office européen des brevets

(11) Numéro de publication: **0 099 766**
**B1**

(12) # FASCICULE DE BREVET EUROPEEN

(45) Date de publication du fascicule du brevet: **09.04.86**

(51) Int. Cl.⁴: **C 07 D 209/34**, A 61 K 31/40

(21) Numéro de dépôt: **83401104.1**

(22) Date de dépôt: **01.06.83**

(54) **Nouveaux dérivés de la 1,3-dihydro 4-(1-hydroxy 2-aminoéthyl) 2H-indol-2-one, leurs sels, procédé de préparation, application à titre de médicaments et compositions les renfermant.**

(30) Priorité: **03.06.82 FR 8209655**

(43) Date de publication de la demande: **01.02.84 Bulletin 84/5**

(45) Mention de la délivrance du brevet: **09.04.86 Bulletin 86/15**

(84) Etats contractants désignés: **AT BE CH DE FR GB IT LI LU NL SE**

(56) Documents cités:
**FR - A - 1 532 210**
**FR - A - 2 370 472**

**HELVETICA CHEMICA ACTA, vol. 51, no. 7, 31 octobre 1968, pages 1616-1628 F. TROXLER et al.: "Synthesen von Indolen mit (2-Aminoäthyl)-,(2-Aminopropyl)- oder Alkanolamin-Seitenketten am Sechsring"**

(73) Titulaire: **ROUSSEL-UCLAF, 35, boulevard des Invalides, F-75007 Paris (FR)**

(72) Inventeur: **Guillaume, Jacques, 15, Avenue du Belvédère Apt. 1904, F-93310-Le Pré-Saint-Gervais (FR)**
Inventeur: **Nedelec, Lucien, 45, boulevard de l'Ouest, F-93340-le Raincy (FR)**
Inventeur: **Brown, Neil Leslie, 12, rue Jaucourt, F-75012-Paris (FR)**
Inventeur: **Plassard, Guy, 6, rue Clément Marot, F-91600-Savigny-sur-Orge (FR)**

(74) Mandataire: **Vieillefosse, Jean-Claude et al, ROUSSEL-UCLAF Boîte postale no. 9 111, route de Noisy, F-93230 Romainville (FR)**

**EP 0 099 766 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne de nouveaux dérivés de la 1,3-dihydro 4-(1-hydroxy-2-amino éthyl) 2H-indol-2-one ainsi que leurs sels, le procédé de préparation, l'application à titre de médicaments de ces nouveaux dérivés et les compositions les renfermant.

L'invention a pour objet de nouveaux dérivés de la 1,3-dihydro 4-(1-hydroxy-2-amino éthyl) 2H indol-2-one ainsi que leurs sels d'addition avec les acides minéraux ou organiques, caractérisés en ce qu'ils répondent à la formule générale I:

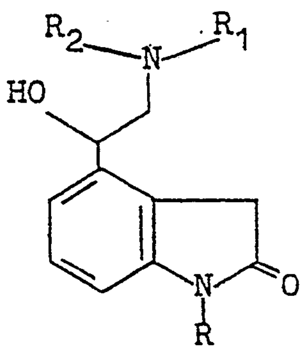

(I)

dans laquelle R représente un atome d'hydrogène ou un radical alcoyle renfermant de 1 à 6 atomes de carbone ou phényl alcoyle renfermant de 7 à 10 atomes de carbone, $R_1$ et $R_2$ représentent un atome d'hydrogène, un radical alcoyle linéaire ou ramifié renfermant de 1 à 8 atomes de carbone, éventuellement substitué, sauf dans la cas du radical méthyl, par un ou plusieurs atomes d'halogène ou par un ou plusieurs radicaux hydroxy, phényle, phénoxy ou phényle ou phénoxy substitués par un ou plusieurs halogènes, hydroxy, méthyl ou méthoxy ou $R_1$ et $R_2$ représentant un radical cycloalcoyle renfermant de 3 à 7 atomes de carbone, un radical cycloalcoylalcoyle renfermant de 4 à 7 atomes de carbone ou un radical allyl ou propargyl.

Dans la formule générale I et dans ce qui suit, le terme radical alcoyle renfermant de 1 à 6 atomes de carbone désigne, de préférence, un radical méthyl, éthyl ou n-propyl; le terme radical phénylalcoyle désigne, de préférence, un radical benzyl ou phénéthyl; le terme radical alcoyle linéaire ou ramifié renfermant de 1 à 8 atomes de carbone, désigne par exemple, un radical méthyl, éthyl, n-propyl, isopropyl, n-butyl isobutyl ou pentyl, et de préférence, un radical méthyl, éthyl propyl, isopropyl ou isobutyl; le radical alcoyle lorsqu'il est substitué, comporte de préférence un seul substituant; lorsque le radical alcoyle est substitué par un ou plusieurs atomes d'halogène. il agit de préférence d'atoms de chlore; le terme radical cycloalcoyle renfermant de 3 à 7 atomes de carbone désigne de préférence, un radical cyclopentyl; le terme radical cycloalcoylalcoyle renfermant de 4 à 7 atomes de carbone désigne, de préférence, un radical cyclopropylméthyl.

Les sels d'addition avec les acides minéraux ou organiques peuvent être par exemple, les sels formés avec les acides chlorhydrique, bromhydrique, iodhydrique, nitrique, sulfurique, phosphorique, acétique, formique, propionique, benzoïque maléique, fumarique, succinique, tartrique, citrique, oxalique, glyoxylique, aspartique, alcanesulfoniques, tels que les acides méthane et éthanesulfoniques, arylsufoniques, tels que les acides benzene et paratoluene sulfoniques, et arylcarboxyliques.

Parmi les produits objet de l'invention, on peut citer notamment les dérivés répondant à la formule I ci-dessus, ainsi que leurs sels d'addition avec les acides minéraux ou organiques, caractérisés en ce que dans ladite formule I, R représente un atome d'hydrogène ou un radical alcoyle renfermant de 1 à 3 atomes de carbone, $R_1$ et $R_2$ représentent un atome d'hydrogène ou un radical alcoyle linéaire ou ramifié renfermant de 1 à 8 atomes de carbone, éventuellement substitué.

Parmi ceux-ci, on peut citer plus particulièrement les dérivés caractérisés en ce que dans ladite formule I, R représente un atome d'hydrogène et $R_1$ et $R_2$ représentent un atome d'hydrogène ou un radical alcoyle linéaire ou ramifié renfermant de 1 à 8 atomes de carbone, éventuellement substitué par un radical phényl ou phénoxy, ces derniers radicaux eux-mêmes éventuellement substitués par un atome d'halogène.

On peut citer tout particulièrement les produits décrits ci-après dans les exemples et parmi ceux-ci,
- la 1,3-dihydro 4-/1-hydroxy 2-(1-méthyléthylamino) éthyl/ 2H-indol-2-one,

ainsi que ses sels d'addition avec les acides minéraux ou organiques.

On peut citer également:
- la 1,3-dihydro 4-/1-hydroxy 2-(3,4-diméthoxy phényl méthyl amino) éthyl/ 2H-indol-2-one,
- la 1,3-dihydro 4-/1-hydroxy 2-(butylamino) éthyl/ 2H-indol-2-one,
- la 1,3-dihydro 4-/1-hydroxy 2-(phénoxyéthylamino) éthyl/ 2H-indol-2-one,

ainsi que leurs sels d'addition avec les acides minéraux ou organiques.

L'invention a également pour objet un procédé de préparation des dérivés tels que définis par la formule I ci-dessus ainsi que de leurs sels, caractérisé en ce que l'on soumet un produit de formule II

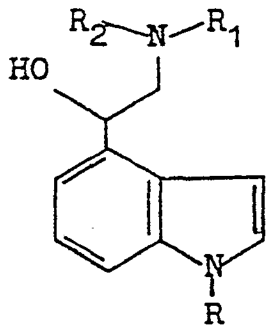

(II)

dans laquelle R, $R_1$ et $R_2$ ont la signification déjà indiquée, à l'action d'un agent d'halogénation, pour obtenir un produit de formule III

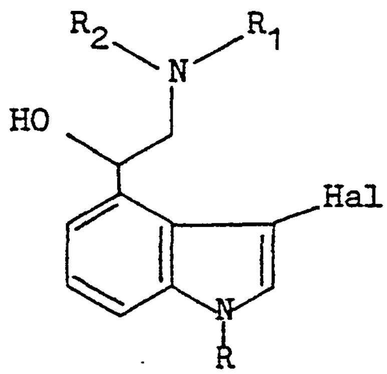

(III)

dans laquelle Hal représente un atome d'halogène et R, $R_1$ et $R_2$ ont la signification déjà indiquée, que l'on hydrolyse pour obtenir un produit de formule I

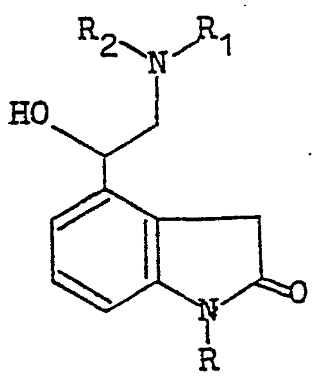

(I)

dans laquelle R, $R_1$et $R_2$ ont la signification déjà indiquée, que l'on isole et, si désiré, salifie.

L'halogénation des produits de formule II peut être réalisée, par exemple, à l'aide du complexe bromé de la pyridine de formule

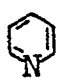

$Br_2$, HBr dans le cas de la bromation. Elle est réalisée avantageusement à l'aide d'un N-halosuccinimide, par exemple le N-bromo ou, de préférence, le N-chlorosuccinimide.

Le produit de formule III obtenu est de préférence un produit chloré.

L'hydrolyse du produit de formule III est réalisée de préférence, à l'aide d'un acide minéral en solution

aqueuse, tel que l'acide phosphorique, l'acide sulfurique ou de preférence l'acide chlorhydrique. Cette solution peut être utilisée concentrée, mais de préférence diluée, par exemple en solution normale.

Les dérivés de formule I présentent un caractère basique. On peut avantageusement préparer les sels d'addition des dérivés de formule I en faisant réagir, en proportions sensiblement stoechiométriques, un acide minéral ou organique avec ledit dérivé de formule I. Les sels peuvent être préparés sans isoler les bases correspondantes.

Les dérivés objet de la présente invention, possèdent de très intéressantes propriétés pharmacologiques; ils sont doués notamment de remarquables propriétés antihypertensives et hypotensives. Certains sont en outre doués de propriétés antiinflammatoires. Certains autres dérivés de formule I sont également doués d'une importante activité antiulcéreuse dans les affections du tractus digestif, ainsi que d'une bonne activité antisécrétoire gastrique. Ces propriétés sont illustrées plus loin dans la partie expérimentale.

Ces propriétés justifient l'utilisation des dérivés de la 1,3-dihydro 4-/1-hydroxy 2-aminoéthyl/ 2H-indol-2-one de formule I, ainsi que de leurs sels pharmaceutiquement acceptables, à titre de médicaments.

La présente demande a ainsi également pour objet l'application, à titre de médicsments, des dérivés de la 1,3-dihydro 4-/1-hydroxy-2-aminoéthyl/ 2H-indol-2-one, tels que définis par la formule I, ainsi que de leurs sels d'addition avec les acides pharmaceutiquement acceptables.

Parmi les médicaments, objet de l'invention, on retient de préférence les médicaments caractérisés en ce qu'ils sont constitués par les nouveaux dérivés de la 1,3-dihydro 4-/1-hydroxy-2-amino éthyl/ 2H indol-2-one répondant à la formule I dans laquelle R représente un atome d'hydrogène ou un radical alcoyle renfermant de 1 à 3 atomes de carbone, $R_1$ et $R_2$ représentent un atome d'hydrogène ou un radical alcoyle linéaire ou ramifié renfermant de 1 à 8 atomes de carbone, éventuellement substitué, ainsi que par leurs sels d'addition avec les acides pharmaceutiquement acceptables.

Parmi ceux-ci, on retient notamment ceux répondant à la formule I, dans laquelle R représente un atome d'hydrogène, et $R_1$ et $R_2$ representent un atome d'hydrogène ou un radical alcoyle lineaire ou ramifié renfermant de 1 à 8 atomes de carbone, éventuellement substitué par un radical phényl ou phénoxy, ces derniers radicaux eux-mêmes éventuellement substitués par un atome d'halogène, ainsi que leurs sels d'addition avec les acides pharmaceutiquement acceptables.

Parmi ces derniers, on retient tout particulierement la 1,3-dihydro 4-/1-hydroxy 2-(1-méthyléthylamino) éthyl/ 2H-indol 2-one, ainsi que ses sels d'addition avec les acides pharmaceutiquement acceptables.

Les médicaments selon l'invention trouvent leur emploi, par exemple dans le traitement de l'hypertension artérielle essentielle, de l'hypertension de la cinquantaine, de la ménopause, du diabétique, de l'obèse et du pléthorique, ainsi que dans le traitement de l'hypertension artérielle du sujet age, ou atteint d'artériosclérose, et dans le traitement de l'hypertension d'origine rénale.

Les médicaments anti-inflammatoires selon l'invention trouvent leur emploi, par exemple, dans le traitement des algies diverses, des affections rhumatismales, des douleurs dentaires, des maladies inflammatoires, notamment des arthroses et des lumbagos.

Les médicaments antiulcéreux selon l'invention trouvent leur emploi, par exemple, dans le traitement des hyperchlorhydries, des ulcères gastriques et gastro-duodénaux, des gastrites, des hernies hiatales, des affections gastriques et grastroduodénales s'accompagnant d'hyperacidité gastrique.

La dose usuelle, variable selon le produit utilisé, le sujet traité et l'affection en cause, peut être par exemple, de 5 mg à 200 mg par jour, par voie orale, chez l'homme, du produit de l'exemple 1 pour le traitement de l'hypertension artérielle essentielle ou de la phase aiguë d'une arthrose. Elle peut être de 5 mg à 200 mg par jour, par voie orale, chez l'homme, du produit de l'exemple 2 pour le traitement de l'ulcère gastrique.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule et leurs sels d'addition avec les acides pharmaceutiquement acceptables peuvent être incorpores dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou vegétale, les dérivés paraffiniques, les glycols, les divers agents mouillants, dispersants ou émulsifiants, les conservateurs.

Le procédé de l'invention permet de préparer des intermédiaires nouveaux, à savoir les produits de formule (III):

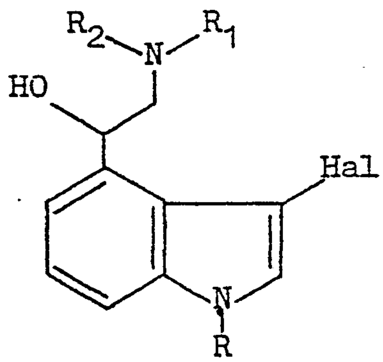

(III)

dans laquelle Hal, R, $R_1$ et $R_2$ ont la signification déjà indiquée.

Les produits de formule II peuvent être préparés comme décrit dans la demande de brevet européen 0 076 713.

D'autres préparations des produits de formule II sont néanmoins possibles. par exemple, les produits de formule $II_B$:

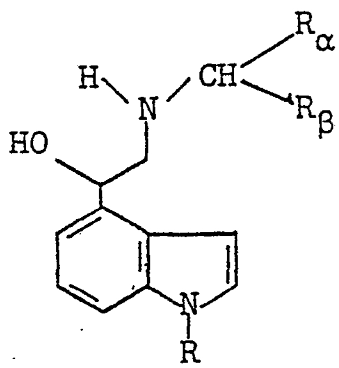

($II_B$)

décrits dans la demande de brevet européen 0 076 713, formule $II_B$ dans laquelle R est défini comme précédemment et le radical

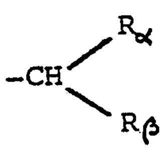

représente un radical alcoyle renfermant de 2 à 8 atomes de carbone, peuvent aussi être préparés par réaction du produit de formule $II_A$:

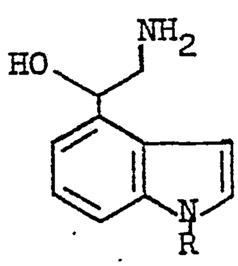

($II_A$)

décrit dans la même demande de brevet eurepéen, avec un halogènure d'acyle, par exemple un chlorure d'acyle tel que d'acétyle ou de propionyle, puis réduction de produit obtenu, par exemple par action d'hydrure d'aluminium-lithium.

Une telle préparation est illustrée ci-après dans la partie expérimentale.

Les exemples qui suivent illustrent la présente invention sans toutefois la limiter:

**EXEMPLE 1: 1,3-dihydro 4-/2-(1-méthyléthyl amino) 1-hydroxy éthyl/ 2H-indol-2-one, et chlorhydrate**

**Stade A: 3-chloro α-/(1-méthyléthyl)amino/méthyl/1H-indol 4-méthanol**

On maintient sous agitation et atmosphère inerte pendant 30 minutes 4,9 g d'α-/(1-méthyléthyl)amino/méthyl/ 1H-indol-4-méthanol dans 110 $cm^3$ d'acide acétique, avec 3,3 g de Nchlorosuccinimide, dilue à l'eau, alcalinise par addition de lessive de soude, extrait à l'acétate d'éthyle, élimine Le solvant par distillation, purifie par chromatographie sur silice (éluant: chloroforme-acétone-triéthylamine 8/1/1) et obtient 4,7 g du produit attendu utilisé tel quel au stade suivant.

**Stade B: 1,3-dihydro 4-/2-(1-méthyléthyl amino)1-hydroxy éthyl/ 2H-indol-2-one et chlorhydrate**

On maintient le produit ci-dessus sous agitation et atmosphere inerte, pendant 20 heures dans 200 $cm^3$ d'acide chlorhydrique 1N, dilue par 200 $cm^3$ d'eau, alcalinise par addition de lessive de soude, extrait à l'acétate d'éthyle, amène à sec sous pression réduite à 500 C et obtient 3,7 g du produit attendu.

**Formation du chlorhydrate:**

On dissout la base ci-dessus dans 10 $cm^3$ d'isopropanol et 40 $cm^3$ d'acétate d'éthyle, ajoute une solution d'acétate d'éthyle saturée d'acide chlorhydrique puis essore les cristaux. On recristallise le chlorhydrate isolé dans 400 $cm^3$ d'isopropanol et 100 $cm^3$ de méthanol et obtient en 2 jets 2,7 g puis 0,6 g du produit attendu (F ≅ 250° C).

Analyse pour $C_{13}H_{18}N_2O_2$, HCl ≙ 270,761

| | | | | |
|---|---|---|---|---|
| Calculé : | C % 57,67 | H % 7,07 | N % 10,35 | Cl % 13,09 |
| Trouvé : | 57,5 | 7,0 | 10,3 | 13,1 |

L'α -/(1-méthyléthyl) amino/ méthyl/ 1H-indol-4-méthanol de départ a été préparé comme décrit dans la demande de brevet européen 0 076 713.

**EXEMPLE 2: 1,3-dihydro 4-/1-hydroxy 2-propylamino)éthyl/ 2H-indol-2-one et son chlorhydrate.**

**Stade A: 3-chloro α-/(1-propyl)amino/méthyl/ 1H-indole-4-méthanol.**

On maintient sous agitation et atmosphère inerte pendant 30 minutes 7,5 g d' α-/(1-propyl)amino/méthyl/ 1H-indole-4-méthanol dans 150 $cm^3$ d'acide acétique, avec 5 g de N-chloro succinimide, dilue par 150 $cm^3$ d'eau, alcalinise par addition de lessive de soude, sature la phase aqueuse de carbonate de potassium, extrait à l'acétate d'éthyle, élimine le solvant, purifie par chromatographie sur silice (éluant: acétate d'éthyle-triéthylamine 9/1) et obtient 7 g du produit attendu utilisé tel quel au stade suivant (Rf = 0,30)

**Stade B: 1,3-dihydro 4-/1-hydroxy 2-(propyl amino)éthyl/ 2H-indol-2-one et son chlorhydrate.**

On maintient le produit ci-dessus sous agitation et atmosphère inerte, pendant 20 heures, dans 300 $cm^3$ d'acide chlorhydrique 1N, alcalinise par addition de lessive de soude sature la phase aqueuse par addition de carbonate de potassium extrait à l'acétate d'éthyle, glace, filtre, amène à sec sous pression reduite et obtient 3,8 g de la base attendue (P ≙ 163°C)

**Formation du chlorhydrate:**

On dissout la base ci-dessus dans 100 $cm^3$ d'isopropanol, ajoute un excès d'isopropanol chlorhydrique à 0° / 5° C, filtre sèche sous pression réduite, recristallise dans 200 $cm^3$ d'éthanol et 100 $cm^3$ de méthanol et obtient en 2

jets 3,3 g puis 0,6 g du produit attendu (F ≃ 258° C)

<u>Analyse:</u> $C_{13}H_{19}N_2ClO_2$ = 270,761

| | | | | |
|---|---|---|---|---|
| Calculé : | C % 57,67 | H % 7,07 | N % 10,35 | Cl % 13,09 |
| Trouvé : | 57,5 | 7,1 | 10,3 | 13,1 |

L'α-/(1-propyl) amino/ méthyl/ 1H-indol-4-méthanol de depart a été préparé comme décrit dans la demande de brevet européen 0 076 713.

**EXEMPLE 3: 1,3-dihydro 4-(1-hydroxy 2-(méthylamino)éthy-1) 2H-indol-2-one et son chlorhydrate**

**Stade A: 3-chloro α-/(1-méthylamino)méthyl/ 1H-indol-4-méthanol.**

On maintient sous agitation et atmosphère inerte pendant 30 minutes 6,5 g d'α-/(1-méthyl)amino/méthyl/ 1H-indol-4-méthanol dans 120 cm$^3$ d'acide acétique, avec 4,6 g de N-chlorosuccinimide, dilue avec 150 cm$^3$ d'eau environ, alcalinise par addition de lessive de soude, extrait à l'acétate d'éthyle, purifie par chromatographie sur silice (éluant: chloroforme methanol-triéthylamine 7/2/1) et obtient 6,1 g de produit brut (Rf = 0,45) utilisé tel quel au stade suivant.

**Stade B: 1,3-dihydro 4-(1-hydroxy 2-(méthyl amino)éthyl/ 2H-indol-2-one et chlorhydrate.**

On maintient le produit ci-dessus sous agitation et atmosphère inerte, pendant 20 heures dans 250 cm$^3$ d'acide chlorhydrique N, alcalinise par addition de lessive de soude, élimine l'eau, reprend l'extrait sec par un mélange chloroforme-méthanol triéthylamine 7/2/1, filtre, purifie par chromatographie sur silice en éluant avec le même mélange de solvants que ci-dessus et obtient 2,4 g de la base attendue, F ≃ 140° C.

**Formation du chlorhydrate:**

On dissout la base ci-dessus dans 200 cm$^3$ d'isopropanol, ajoute un excès d'isopropanol chlorhydrique, filtre, sèche sous pression réduite, recristallise dans 200 cm$^3$ d'éthanol et 100 cm$^3$ de méthanol et obtient 2 g du produit attendu F ≃ 258° C avec décomposition

<u>Analyse</u> : $C_{11}H_{15}N_2ClO_2$ = 242,708

| | | | | |
|---|---|---|---|---|
| Calculé : | C % 54,44 | H % 6,23 | N % 11,54 | Cl % 14,61 |
| Trouvé : | 54,2 | 6,4 | 11,2 | 14,7 |

L'α- (méthylamino) méthyl 1H-indol-4-méthanol de départ a été préparé comme décrit dans la demande de brevet européen 0 076 713.

**EXEMPLE 4: Chlorhydrate de 1,3-dihydro 4-/(1-hydroxy 2-amino) éthyl/ 2H -indol-2-one.**

**Préparation: α-(trifluoro acétamidométhyl) 1H-indol-4-méthanol.**

On agite à 0° C pendant 1 heure et sous atmosphère inerte, 3,11 g de trifluorothioacétate d'éthyle dans 2 cm$^3$ de diméthylformamide et 3,5 g d'α-(aminométhyl) 1H-indol-4-méthanol dans 20 cm$^3$ de diméthylformamide contenant un équivalent de triéthylamine. On verse le mélange réactionnel dans l'eau extrait à l'éther, lave à l'eau la phase organique, concentre à sec et reprend le résidu par du chloroforme. On essore les cristaux formés et obtient 4,66 g de produit attendu.

**Stade A: 3-chloro α-(trifluoroacétamidométhyl) 1H-indol 4-méthanol.**

On agite à température ambiante, pendant 30 minutes, 7,4 g de produit obtenu à la préparation ci-dessus, dans 120 $cm^3$ d'acide acétique avec 4 g de N-chlorosuccinimide. On dilue à l'eau, alcalinise par addition d'ammoniaque concentrée, sature de carbonate de potassium et extrait à l'acétate d'éthyle. On concentre à sec et chromatographie le résidu sur silice en éluant avec un mélange benzène-acétate d'éthyle (7-3). On isole les fractions de rf:0,35, et obtient 6,95 g de produit attendu.

**Stade B: Chlorhydrate de 1,3-dihydro 4-/(1-hydroxy 2-amino) éthyl/ 2H-indol-2-one.**

On agite 6,95 g du produit obtenu ci-dessus et 350 $cm^3$ d'acide chlorhydrique N pendant 24 heures, à température ambiante, puis chauffe au reflux pendant 1 heure. On refroidit à 0,+5°C et alcalinise par addition de lessive de soude, puis chasse l'eau sous pression réduite à 50°C. On reprend le résidu par un mélange chloroforme-méthanol (1-1), filtre et concentre à sec sous pression réduite. On chromatographie le résidu sur silice avec un mélange chloroforme-méthanol-triéthylamine (7-2-1) et obtient 800 mg de produit que l'on dissout dans 70 $cm^3$ d'isopropanol et ajoute un excès d'une solution d'isopropanol saturée en acide chlorhydrique. Après concentration à 35 $cm^3$ environ, on glace, essore et obtient 633,3 mg de produit attendu. On recristallise le produit. dans un mélange acétonitrile-méthanol (6-1). F ≃ 270°C.

| | | | | | |
|---|---|---|---|---|---|
| <u>Analyse</u> : | $C_{10}H_{12}N_2O_2$ = 228,680 | | | | |
| Calculé : | C% 52,52 | H% 5,73 | N% 12,25 | Cl% 15,5 |
| Trouvé : | 52,8 | 5,7 | 12,4 | 15,4 |

**EXEMPLE 5: Chlorhydrite de 1,3-dihydro 4-/1-hydroxy 2-(cyclopropylméthylamino) éthyl/ 2H-indol-2-one.**

**Préparation:**

**Stade a:**

α-(cyclopropylcarbonylaminométhyl) 1H-indol-4-méthanol.

On refroidit à 0,+5°C une solution contenant 5,6 g dα-(ami-nométhyl) 1H-indol 4-méthanol dans 100 $cm^3$ de tétrahydrofuranne et 8,9 $cm^3$ de triéthylamine, ajoute 3,6 $cm^3$ de chlorure de l'acide cyclopropane carboxylique dane 30 $cm^3$ de tétrahydrofuranne et agite 30 minutes à cette température. On dilue à l'eau, extrait à l'acétate d'éthyle, lave à l'eau saturée en chlorure de sodium, sèche et concentre à sec sous pression réduite. On reprend le résidu à l'éther et obtient 6,7 g de produit attendu. F = 158°C.

**Stade b:** α-(cyclopropylméthylaminométhyl) 1H-indol-4-méthanol.

On ajoute lentement 5,2 g d'hydrure de lithium et d'aluminium à 6,7 g de produit obtenu ci-dessus dans 200 $cm^3$ de tétrahydrofuranne et chauffe au reflux pendant 1 heure. On refroidit à 0,+5°C, détruit l'excès d'hydrure par addition de tétrahydrofuranne à 20% d'eau, puis à l'eau et à l'acétate d'éthyle, filtre l'insoluble, décante et poursuit l'extraction à l'acétate d'éthyle. On lave les phases organiques réunies à l'eau saturée en chlorure de sodium, sèche et évapore les solvants sous pression réduite. On chromatographie le résidu sur silice, élue par un mélange chloroforme-méthanol-triéthylamine (8-1-1) et recueille 5,5 g de produit attendu. rf:0,45.

**Stade A: 3-chloro α-(cyclopropylméthylaminométhyl) 1H-indol-4-méthanol.**

On opère comme au stade A de l'exemple 4 en partant de 3,7 g du produit obtenu à la préparation ci-dessus, 80 $cm^3$ d'acide acétique et 2,4 g de N-chlorosuccinimide en agitant 1 heure. Après chromatographie du résidu sur silice en éluant par un mélange chloroforme-méthanol-triéthylamine (8-1-1), on obtient 3,35 g de produit attendu. rf: 0,50.

**Stade B: Chlorhydrate de 1,3-dihydro 4-/1-hydroxy 2-(cyclopropylméthylamino) ethyl/ 2H-indol-2-one.**

On opère comms au stade B de l'exemple 4 en partant de 3,35 g de produit obtsnu ci-dessus et 100 $cm^3$ d'acide chlorhydrique N, en agitant 20 heures à température ambiante sans chauffage. Après alcalinisation à la lessive ds soude, on sature par addition de carbonate de sodium et extrait à l'acétate d'éthyle. On concentre à sec et reprend le résidu par l'éther. On obtient 2,7 g de produit. F = 163° Ç. On dissout ce dernier dans 100 $cm^3$ d'acétate d'éthyle et 50 $cm^3$ de méthanol et ajoute une solution d'acétats d'éthyle saturée d'acide chlorhydrique. On obtient 2,3 g de produit attendu. F = 265° C.

| | | | | | |
|---|---|---|---|---|---|
| Analyse | : $C_{14}H_{18}N_2O_2H\ Cl$ | | | | |
| Calculé | : | C% 59,47 | H% 6,77 | N% 9,91 | Cl% 12,54 |
| Trouvé | : | 59,2 | 6,8 | 9,6 | 12,6 |

**EXEMPLE 8: 1,3-dihydro 4-/(1-hydroxy 2-dipropylaminol- éthyl/ 2H-indol 2-one et son tartrate neutre.**

**Stade A: 3-chloro α-(dipropylaminométhyl) 1H-indol-4-méthanol.**

On opère comme au stade A de l'exemple 4 à partir de 5,62 g d'α-(dipropylaminométhyl) 1H-indol-4-méthanol, 100 $cm^3$ d'acide acétique et 3,25 g de N-chlorosuccinimide en agitant 1 heure. Après chromatographie sur silice en éluant par un mélange cyclohexane-chloroforme-triéthylamine (6-3-1), on obtient 6,1 g de produit attendu, rf = 0,30.

**Stade B: 1,3-dihydro 4-/(1-hydroxy 2-dipropylamino) éthyl/ 2H-indol-2-one et son tartrate neutre.**

On opère comme au stade B de l'exemple 4 à partir de 6,1 g du produit obtenu ci-dessus en laissant 20 heures à température ambiante sans chauffage. On chromatographie le résidu sur silice en éluant par un mélange cyclohexane-chloroforme-triéthylamine (6-3-1) et recueille 4,4 g de produit. F = 95°C. On dissout ce dernier dans 200 $cm^3$ d'isopropanol et ajoute 2,4 g d'acide tartrique. On chauffe au reflux. Après refroidissement, on essore et obtient 4 g de produit attendu. F = 215°C.

| | | | | |
|---|---|---|---|---|
| Analyse | : $C_{36}H_{54}N_4O_{10}$ | | | |
| Calculé | : | C% 61,52 | H% 7,74 | N% 7,97 |
| Trouvé | : | 61,3 | 7,7 | 8,0 |

**EXEMPLE 7: Chlorhydrate de 1,3 -dihydro 4/(1-hydroxy -2-éthylamino) éthyl/ 2H -indol-2-one.**

**Preparation:**

**Stade a:**

α-(acétaminométhyl) 1H-indol-4-méthanol.

On refroidit à -5,-10° C, 3,04 g dα-(aminométhyl) 1H-indol-4-méthanol dans 30 $cm^3$ de tétrahydrofuranne 4,6 $cm^3$ de triié thylamine. On ajoute 1,83 $cm^3$ de chlorure d'acétyle et agite 30 minutes à cette température. On dilue à l'eau, extrait à l'acétate d'éthyle, lave à l'eau saturée de chlorure de sodium, seche, concentre à sec et obtient 4,32 g de résidu qus l'on reprend par un mélange chloroforme-méthanol (9-1), et isole 0,762 g de produit attendu. F = 99°C.

**Stade b:**

α-(éthylaminométhyl) 1H-indol-4-méthanol.

On opère comme au stade b de la préparation pour l'exemple 5, à partir de 2,84 g de produit préparé comme ci-dessus dans 100 $cm^3$ de tétrahydrofuranne et 5 g d'hydrure de lithium et d'aluminium, à +5, +10°C puis au reflux jusqu'à réaction complete. On chromatographie le résidu obtenu sur silice, élue par un mélange chloroforme-méthanol-triéthylamine (90-5-5) et isole 2,35 g de produit attendu.

**Stade A: 3-chloro α-(éthylaminométhyl) 1H-indol-4-méthanol.**

On opère comme au stade A de l'exemple 4 à partir de 5,18 g de produit obtenu à la préparation ci-dessus dans 100 $cm^3$ d'acide acétique et 3,73 g de N-chlorosuccinimide et agite pendant 20 minutes. On chromatographie le résidu sur silice en éluant par un mélange chloroforme-méthanol-triéthylamine (8-1-1) et obtient 3,38 g de produit attendu.

**Stade B: Chlorhydrate de 1,3-dihydro 4-/(1-hydroxy 2-éthylamino) éthyl/ 2H-indol-2-one.**

On agite à température ambiante, pendant 19 heures, 3,38 g du produit obtenu précédemment dans 100 $cm^3$ d'acide chlorhydrique N. On filtre l'insoluble, glace le filtrat, alcalinise par addition de 10 $cm^3$ de lessive de soude et sature avec du carbonate de potassium. On extrait à l'acétate d'éthyle, lave à l'eau saturée de chlorure de sodium, sèche, concentre à sec et obtient 2,4 g de produit que l'on chromatographie sur silice en éluant par un mélange chloroforme-méthanol-triéthylamine (8-1-1) pour obtenir 1,95 g de produit. F = 166°C. On dissout ce dernier dans 40 $cm^3$ d'isopropanol, ajoute une solution d'isopropanol saturée d'acide chlorhydrique, glace et agite pendant 1 heure et demie. On recueille 2,22 g de chlorhydrate qui, recristallisé dans un mélange éthanol-méthanol (27-19) donne 1,73 g de produit pur attendu. F = 263-265°C.

Analyse : $C_{12}H_{16}N_2O_2$, H Cl

| | | | | |
|---|---|---|---|---|
| Calculé : | C% 56,14 | H% 6,67 | N% 10,91 | Cl% 13,80 |
| Trouvé : | 56,1 | 6,7 | 10,9 | 13,8 |

**EXEMPLE 8:**

On a préparé des comprimés répondant à la formule:
- Chlorhydrate de 1,3-dihydro 4-/(1-hydroxy 2-(1-méthyl éthylamino) éthyl/2H-indol-2-one.................... 10 mg
-Excipient q.s. pour un comprimé terminé à........... 100 mg
(détail de l'excipient: lactose, amidon, talc, stéarate de magnésium).

**EXEMPLE 9:**

On a préparé des comprimés répondant à la formule:
- Chlorhydrate de 1,3-dihydro 4-/1-hydroxy 2-(propyl amino)éthyl/ 2H-indol-2-one........................ 20 mg
- Excipient q.s. pour un comprimé terminé à.......... 100 mg
(détail de l'excipient: lactose, amidon, talc, stéarate de magnésium).

**Etude pharmacologique:**

1°) Détermination de l'activité hypotensive:

L'activité hypotensive a été étudiée sur des rats mâles de souche WISTAR pesant 300 g environ et anesthésiés au nembutal (50 mg/kg par voie intraveineuse).

Le produit testé a été administré par voie intraveineuse dans la veine pénile.

La pression artérielle carotidienne a été mesurée avant et après administration du produit testé.

Le tableau ci-après indique les variations exprimées en pourcentage de la pression artérielle après administration du produit testé par rapport à la pression artérielle témoin initiale.

| Produits de l'exemple | Dose mg/kg | Variation % de la pression artérielle | | | |
|---|---|---|---|---|---|
| | | 1 minute après l'administration | 5 minutes après l'administration | 10 minutes après l'administration | 30 minutes après l'administration |
| 1 | 1 | - 54 | - 45 | - 36 | - 32 |
| | 0,1 | - 36 | - 31 | - 29 | - 27 |
| 2 | 1 | - 40 | - 39 | - 41 | - 36 |
| | 0,1 | - 14 | - 11 | - 13 | - 20 |
| 3 | 0,1 | - 13 | - 10 | - 12 | - 13 |
| 5 | 0,01 | - 35 | - 24 | - 22 | - 21 |
| 7 | 0,01 | - 21 | - 13 | - 13 | - 12 |

**2°) Détermination de l'activité antiulcéreuse.**

**Méthode:**

L'essai est effectué selon la méthode de Shay modifiée par GUTH (GUTH, p.H. et coll, Topical aspirin plus HCl gastric lesions in the rat. Gastroenterology 1979,76, 88-93.) Des rats mâles Sprague-Dawley d'un poids voisin de 250 g,à jeun depuis 48 heures sauf boisson, ont été utilisés. On pratique sous légère anesthésie à l'halothane, la ligature du pylore et une heure plus tard, on administre l'aspirine per os à la dose de 200 mg/kg, en suspension dans la carboxyméthylcellulose à 0,25% renfermant 15% d'acide chlorhydrique N. Deux heures plus tard, on sacrifie les animaux, prélève et examine les estomacs. Chaque lésion ulcéreuse est cotée de la fagon suivante:

0,5: ulcère < 0,5 mm
1: ulcère 0,5 à 1 mm
2: ulcère 1 à 2 mm
3: ulcère 2 à 3 mm.

La somme des cotations est calculée pour chaque estomac, et le score moyen est établi pour chaque lot.

Les substances à étudier sont administrées per os uns heure avant la ligature.

A la dose de 0,4 mg/kg, le produit de l'exemple 2 protège les animaux traités de 60%.

**3°) Détermination de l'activité anti-inflammatoire.**

L'activité anti-inflammatoire a été déterminée sur le test de l'oédème plantaire provoqué par la carraghénine chez le rat.

On administre, à des rats màles pesant de 130 à 150 g, 0,05 $cm^3$ d'une suspension stérile à 1% de carraghénine dans l'articulation tibio-tarsienne d'une patte postérieure.

Simultanément, on administre le produit à étudier dans une suspension de carboxyméthylcellulose à 0,25% et de Tween à 0,02% par voie orale.

Le volume de la patte est mesuré avant l'administration, puis 2 heures, 4 heures, 6 heures, 8 heures et 24 heures après.

L'intensité de l'inflammation est maxima 4 à 6 heures après l'injection de carraghénine. La différence du volume des pattes des animaux traités et des témoins met en évidence l'action anti inflammatoire du médicament.

A la dose de 0,4 mg/kg, le produit de l'exemple 1 protège de 61% les animaux traités.

**4°) Etude de la toxicité aigüe.**

On a évalué les doses létales $DL_0$ des différents composes testés après administration par voie orale chez la souris.

On appelle $DL_0$ la dose maximale ne provoquant aucune mortalité en 8 jours.

Les résultats obtenus sont les suivants:

| Produits de l'exemple | $DL_0$ en mg/kg |
|---|---|
| 1 | 200 |
| 2 | 200 |
| 3 | > 400 |
| 5 | 200 |
| 7 | > 400 |

**Revendications** pour les Etants contractants: BE CH DE FR GB IT LI LU NL SE

1°) Dérivés de la 1,3-dihydro 4-(1-hydroxy-2-amino éthyl) 2H-indol-2-one, ainsi que leurs sels d'addition avec les acides minéraux ou organiques, caractérisés en ce qu'ils répondent à la formule générale I

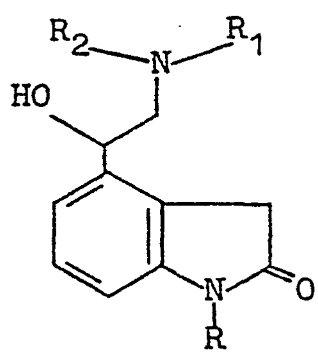

(I)

dans laquelle R représente un atome d'hydrogène ou un radical alcoyle renfermant de 1 à 6 atomes de carbone ou phényl alcoyle renfermant de 7 à 10 atomes de carbone, $R_1$ et $R_2$ représentent un atome d'hydrogène, un radical alcoyle linéaire ou ramifié renfermant de 1 à 8 atomes de carbone, éventuellement substitué, sauf dans le cas du radical méthyl, par un ou plusieurs atomes d'halogène ou par un ou plusieurs radicaux hydroxy, phényle, phénoxy, ou phényls ou phénoxy substitués par un plusieurs halogènes, hydroxy, méthyl ou méthoxy, ou $R_1$ et $R_2$ représentent un radical cycloalcoyle renfermant de 3 à 7 atomes de carbone, un radical cycloalcoylalcoyle renfermant de 4 à 7 atomes de carbone ou un radical allyl ou propargyl.

2°) Dérivés de la 1,3-dihydro 4-(1-hydroxy-2-amino éthyl) 2H-indol-2-one tels que définis par la formule I de la revendication 1, ainsi que leurs sels d'addition avec les acides minéraux ou organiques, caractérisés en ce que R

représente un atome d'hydrogène ou un radical alcoyle renfermant de 1 à 3 atomes de carbone, $R_1$ et $R_2$ représentent un atome d'hydrogène ou un radical alcoyle linéaire ou ramifié renfermant de 1 à 8 atomes de carbone, éventuellement substitué.

3°) Dérivés de la l,3:dihydro 4-(1-hydroxy 2-amino éthyl) 2H-indol-2-one, selon la revendication 2, ainsi que leurs sels d'addition avec les acides minéraux ou organiques, caractérisés en ce que R représente un atome d'hydrogène, et $R_1$ et $R_2$ représentent un atome d'hydrogène ou un radical alcoyle linéaire ou ramifié renfermant de 1 à 8 atomes de carbone, éventuellement substitué par un radical phényl ou phénoxy, ces derniers radicaux eux-mêmes éventuellement substitués par un atome d'hydrogène.

4°) La 1,3-dihydro 4-(1-hydroxy 2-(1-méthyléthylamino) éthyl/ 2H-indol-2-one, ainsi que ses sels d'addition avec les acides minéraux ou organiques.

5°) Procédé de préparation des dérivés répondant à la formule I de la revendication 1, ainsi que de leurs sels d'addition avec les acides minéraux ou organiques, caractérisé en ce que l'on soumet un produit de formule II:

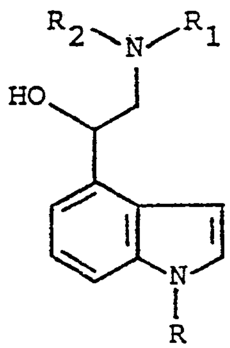

(II)

dans laquelle R, $R_1$ et $R_2$ ont la signification déjà indiquée dans la revendication 1, à l'action d'un agent d'halogénation, pour obtenir un produit de formule III:

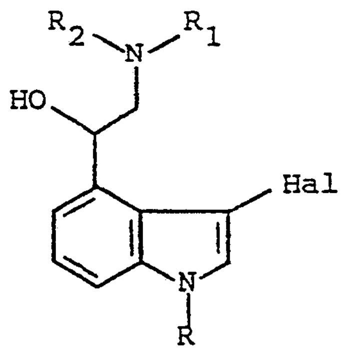

(III)

dans laquelle Hal représente un atome d'halogène et R, $R_1$ et $R_2$ ont la signification déjà indiquée, que l'on hydrolyse pour obtenir un produit de formule I:

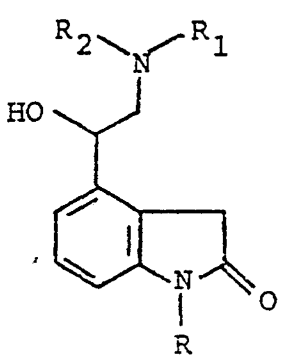

(I)

dans laquelle R, $R_1$ et $R_2$ ont la signification déjà indiqués, que l'on isole et, si désiré, salifie.

6°) Procédé selon la revendication 5, caractérisé en ce que

- l'agent d'halogénation est un N-halosuccinimide,

- l'hydrolyse est réalisée à l'aide d'un acide minéral en solution aqueuse.

7°) Médicaments, caractérisés en ce qu'ils sont constitués par les nouveaux dérivés de la 1,3-dihydro 4-(1-hydroxy-2-aminoéthyl) 2H-indol-2-one, tels que définis par la formule I de la revendication 1, ainsi que par leurs sels d'addition avec les acides pharmaceutiquement acceptables.

8°) Médicaments, caractérisés en ce qu'ils sont constitués par les nouveaux dérivés de la 1,3-dihydro 4-(1-hydroxy 2-aminoéthyl) 2H-indol-2-one, tels que définis dans la revendication 2 ou 3, ainsi que par leurs sels d'addition avec les acides pharmaceutiquement acceptables.

9°) Médicaments, caractérisés en ce qu'ils sont constitués par le dérivé de la 1,3-dihydro 4- (1-hydroxy-2-aminoéthyl) 2H-indol-2-one, tel que défini à la revendication 4, ainsi que par ses sels d'addition avec les acides pharmaceutiquement acceptables.

10°) Compositions phsrmaceutiques, caractérisées en ce qu'elles renferment, à titre de principe actif, 1,un au moins des médicaments, tels que définis à l'une des revendications 7, 8 ou 9.

**Revendications** pour l'Etat contractant:AT

1.- procédé pour préparer les nouveaux dérivés de la 1,3-dihydro 4-(1-hydroxy 2-aminoéthyl) 2H-indol-2-one, ainsi que leurs sels d'addition avec les acides minéraux ou organiques répondant à la formule générale (I):

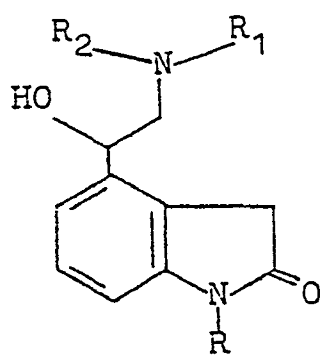

(I)

dans laquelle R représente un atome d'hydrogène ou un radical alcoyle renfermant de 1 à 6 atomes de carbone ou phényl alcoyle renfermant de 7 à 10 atomes de carbone, $R_1$ et $R_2$ représentent un atome d'hydrogène, un radical alcoyle linéaire ou ramifié renfermant de 1 à 8 atomes de carbone, éventuellement substitué, sauf dans le cas du radical méthyl, par un ou plusieurs atomes d'halogène ou par un ou plusieurs radicaux hydroxy, phényle, phénoxy, ou phényle ou phénoxy substitués par un ou plusieurs halogènes, hydroxy, méthyl ou méthoxy, ou $R_1$ et $R_2$ représentent un radical cycloalcoyle renfermant de 3 à 7 atomes de carbone, un radical cycloalcoylalcoyle renfermant de 4 à 7 atomes de carbone ou un radical allyl ou propargyl, caractérisé en ce que l'on soumet un produit de formule (II):

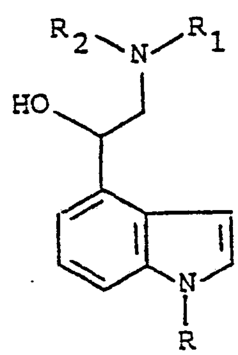

(II)

dans laquelle R, $R_1$ et $R_2$ ont la signification déjà indiquée, à l'action d'un agent d'halogénation, pour obtenir un produit de formule III:

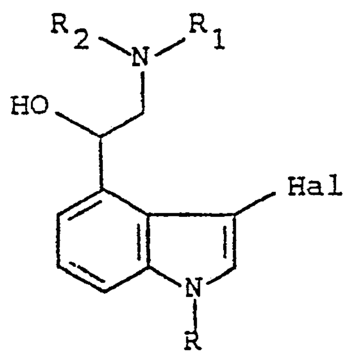

(III)

dans laquelle Hal représente un atome d'halogène et R, $R_1$ et $R_2$ ont la signification déjà indiquée, que l'on hydrolyse pour obtenir un produit de formule I:

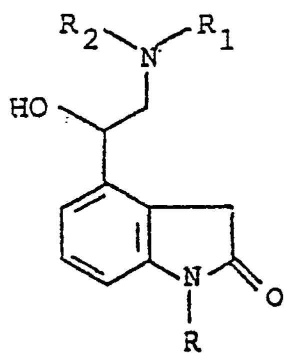

(I)

dans laquelle R, $R_1$ et $R_2$ ont lasignification déjà indiquée, que l'on isole et, si désiré, salifie.

2°) Procédé selon la revendication 1, caractérisé en ce que
- l'agent d'halogenation est un N-halosuccinimide,
- l'hydrolyse est realisée à l'aide d'un acide minéral en solution aqueuse.

3.- Procede selon la revendication 1 ou 2, caractérisé en ce que l'on utilise au départ un produit de formule II dans laquelle R représente un atome d'hydrogène ou un radical alcoyle renfermant de 1 à 3 atomes de carbone, $R_1$ et $R_2$ représentent un atome d'hydrogène ou un radical alcoyle lineaire ou ramifié renfermant de 1 à 8 atomes de carbone, éventuellement substitue.

4.- Procédé selon la revendication 1 ou 2, caractérisé en ce que l'on utilise au départ un produit de formule II dans laquelle R représente un atome d'hydrogène, et $R_1$ et $R_2$ représentent un atome d'hydrogène ou un radical alcoyle linéaire ou ramifié renfermant de 1 à 8 atomes de carbone, éventuellement substitué par un radical phényl ou phénoxy, ces derniers radicaux eux-mêmes éventuellement substitués par un atome d'halogène.

5.- procédé selon la revendication 1 ou 2, caractérisé en ce que l'on prépare la 1,3-dihydro 4-/1-hydroxy 2-(1-méthyléthylamino) éthyl/ 2H-indol-2-one, ainsi que ses sels d'addition avec les acides minéraux ou organiques.

**Patentansprüche** für die Vertragsstaaten: BE, CH, DE, FR, GB, IT, LI, LU, NL,SE

1. 1,3-Dihydro-4-(1-hydroxy-2-aminoethyl)-2H-indol-2-on-Derivate sowie deren Additionssalze mit Mineral-oder organischen Säuren, dadurch gekennzeichnet, daß sie der allgemeinen Formel I

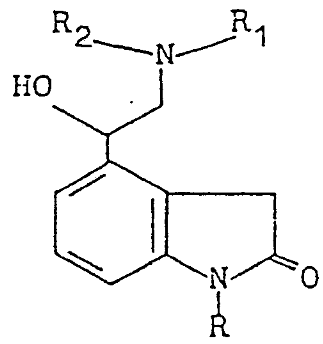

(I)

entsprechen, worin R ein Wasserstoffatom oder einen Alkylrest mit 1 bis 6 Kohlenstoffatomen oder einen Phenylalkylrest mit 7 bis 10 Kohlenstoffatomen bedeutet, $R_1$ und $R_2$ ein Wasserstoffatom, einen linearen oder verzweigten Alkylrest mit 1 bis 8 Kohlenstoffatomen, der gegebenenfalls mit Ausnahme des Methylrestes substituiert ist durch ein oder mekrere Halogenatome oder durch einen oder mehrere Hydroxy-, Phenyl-, Phenoxyreste oder durch Phenyl- oder Phenoxyreste, die durch ein oder mehrere Halogene, Hydroxy-, Methyl- oder Methoxygruppen substituiert sind, bedeuten oder $R_1$ und $R_2$ einen Cycloalkylrest mit 3 bis 7 Kohlenstoffatomen, einen Cycloalkalkylrest mit 4 bis 7 Kohlenstoffatomen oder einen Allyloder Propargylrest darstellen.

2. 1,3-Dihydro-4-(1-hydroxy-2-aminoethyl)-2H-indol-2-on-Derivate der Formel I gemäß Anspruch 1 sowie deren Additionssalze mit Mineral- oder organischen Säuren, dadurch gekennzeichnet, daß R ein Wasserstoffatom oder einen Alkylrest mit 1 bis 3 Kohlenstoffatomen bedeutet, $R_1$ und $R_2$ ein Wasserstoffatom oder einen linearen oder verzweigten Alkylrest mit 1 bis 8 Kohlenstoffatomen, der gegebenenfalls substituiert ist, darstellen.

3. 1,3-Dihydro-4-(1-hydroxy-2-aminoethyl)-2H-indol-2-on-Derivate gemäß Anspruch 2 sowie deren Additionssalze mit Mineral- oder organischen Säuren, dadurch gekennzeichnet, daß R ein Wasserstoffatom bedeutet und $R_1$ und $R_2$ ein Wasserstoffatom oder einen linearen oder verzweigten Alkylrest mit 1 bis 8 Kohlenstoffatomen, der gegebenenfalls durch einen Phenyl- oder Phenoxyrest substituiert ist, darstellen, wobei diese letztgenannten Reste ihrerseits durch ein Halogenatom substituiert sein können.

4. 1,3-Dihydro-4-[1-hydroxy-2-(1-methylethylamino)-ethyl]-2H-indol-2-on und dessen Additionssalze mit Mineral- oder organischen Säuren.

5. Verfahren zur Herstellung der Derivate der Formel I gemäß Anspruch 1 sowie von deren Additionssalzen mit Mineral- oder organischen Säuren, dadurch gekennzeichnet, daß man ein Produkt der Formel II

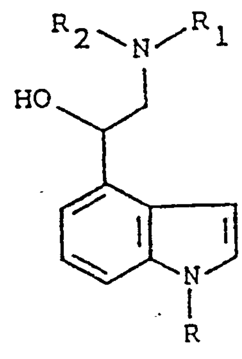

(II)

worin R, $R_1$ und $R_2$ die in Patentanspruch 1 angegebene Bedeutung besitzen, der Einwirkung eines Halogenierungsmittels unterzieht, um ein Produkt der Formel III

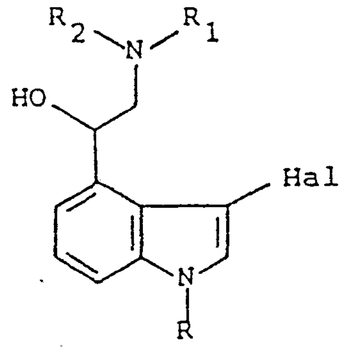

(III)

zu erhalten, worin Hal ein Halogenatom bedeutet und R, $R_1$ und $R_2$ die angegebene Bedeutung besitzen, welches man hydrolysiert, um ein Produkt der Formel I

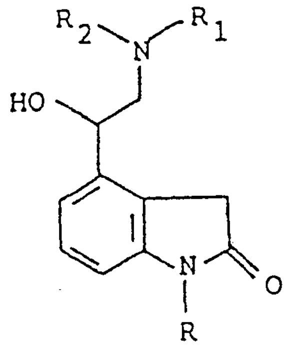

(I)

zu erhalten, worin R, $R_1$ und $R_2$ die angegebene Bedeutung besitzen, welches man isoliert und gewünschtenfalls in ein Salz überführt.

6. Verfahren gemäß Anspruch 5, dadurch gekennzeichnet, daß
das Halogenierungsmittel N-Halosuccinimid ist,
die Hydrolyse mit einer Mineralsäure in wäßriger Lösung durchgeführt wird.

7. Arzneimittel, dadurch gekennzeichnet, daß sie aus den neuen 1,3-Dihydro-4-(1-hydroxy-2-aminoethyl)-2H-indol-2-on-Derivaten der Formel I gemäß Anspruch 1 sowie aus deren Additionssalzen mit pharmazeutisch verträglichen Säuren bestehen.

8. Arzneimittel, dadurch gekennzeichnet, daß sie aus den neuen 1,3-Dihydro-4-(1-hydroxy-2-aminoethyl)-2H-indol-2-on-Derivaten gemäß Anspruch 2 oder 3 sowie aus deren Additionssalzen mit pharmazeutisch verträglichen Säuren bestehen.

9. Arzneimittel, dadurch gekennzeichnet, daß sie aus dem 1,3-Dihydro-4-(1-hydroxy-2-aminoethyl)-2H-indol-2-on-Derivat gemäß Anspruch 4 sowie aus dessen Additionssalzen mit pharmazeutisch verträglichen Säuren bestehen.

10. Pharmazeutische Zusammensetzungen, dadurch gekennzeichnet, daß sie als Wirkstoff zumindest eines der Arzneimittel gemäß einem der Ansprüche 7, 8 oder 9 enthalten.

**Patentansprüche** für den Vertragsstaat: AT

1. Verfahren zur Herstellung von neuen 1,3-Dihydro-4-(1-hydroxy-2-aminoethyl)-2H-indol-2-on-Derivaten sowie von deren Additionssalzen mit Mineral- oder organischen Säuren der allgemeinen Formel (I)

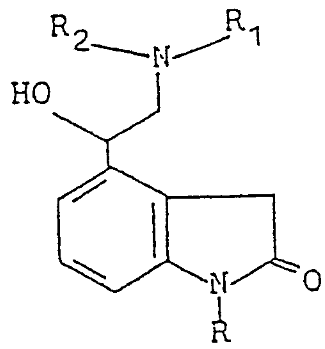

(I)

worin R ein Wasserstoffatom oder einen Alkylrest mit 1 bis 6 Kohlenstoffatomen oder einen Phenylalkylrest mit 7 bis 10 Kohlenstoffatomen bedeutet, $R_1$ und $R_2$ ein Wasserstoffatom, einen linearen oder verzweigten Alkylrest mit 1 bis 8 Kohlenstoffatomen, der gegebenenfalls mit Ausnahme des Methylrestes substituiert ist durch ein oder mehrere Halogenatome oder durch einen oder mehrere Hydroxy-, Phenyl-, Phenoxyreste oder durch Phenyl-oder Phenoxyreste, die substituiert sind durch ein oder mehrere Halogenatome, Hydroxy-, Methyl- oder Methoxygruppen, bedeuten oder $R_1$ und $R_2$ einen Cycloalkylrest mit 3 bis 7 Kohlenstoffatomen, einen Cycloalkalkylrest mit 4 bis 7 Kohlenstoffatomen oder einen Allyl- oder Propargylrest bedeuten, dadurch gekennzeichnet, daß man ein Produkt der Formel (II)

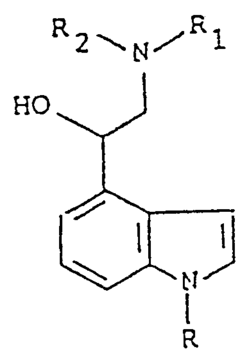

(II)

worin R, $R_1$ und $R_2$ die angegebene Bedeutung besitzen, der einwirkung eines Halogenierungsmittels unterzieht, um ein Produkt der Forme III

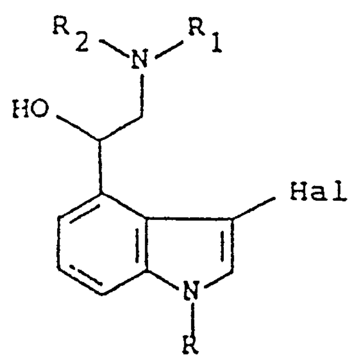

(III)

zu erhalten, worin Hal ein Halogenatom bedeutet und R, $R_1$ und $R_2$ die angegebene Bedeutung besitzen, welches man hydrolysiert, um ein Produkt der Formel I

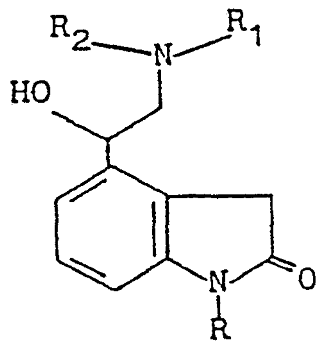

(I)

zu erhalten, worin R, $R_1$ und $R_2$ die angegebene Bedeutung besitzen, welches man isoliert und gewünschtenfalls in ein Salz überführt.

2. Verfahren gemäß Anspruch 1, dadurch gekennzeichnet, daß
das Halogenierungsmittel n-Halosuccinimid ist,
die Hydrolyse mit Hilfe einer Mineralsäure in wäßriger Lösung durchgeführt wird.

3. Verfahren gemäß Anspruch 1 oder 2, dadurch gekennzeichnet, daß man von einem Produkt der Formel II ausgeht, worin R ein Wasserstoffatom oder einen Alkylrest mit 1 bis 3 Kohlenstoffatomen bedeutet, $R_1$ und $R_2$ ein Wasserstoffatom oder einen linearen oder verzweigten Alkylrest mit 1 bis 8 Kohlenstoffatomen, der gegebenenfalls substituiert ist, darstellen.

4. Verfahren gemäß Anspruch 1 oder 2, dadurch gekennzeichnet, daß man von einem Produkt der Formel II ausgeht, worin R ein Wasserstoffatom bedeutet und $R_1$ und $R_2$ ein Wasserstoffatom oder einen linearen oder verzweigten Alkylrest mit 1 bis 8 Kohlenstoffatomen, der gegebenenfalls durch einen Phenyl- oder Phenoxyrest substituiert ist, darstellen, wobei diese letztgenannten Reste ihrerseits gegebenenfalls durch ein Halogenatom substituiert sein können.

5. Verfahren gemäß Anspruch 1 oder 2, dadurch gekennzeichnet, daß man das 1,3-Dihydro-4-[1-hydroxy-2-(1-methylethylamino)-ethyl]-2H-indol-2-on sowie dessen Additionssalze mit Mineral- oder organischen Säuren herstellt.

**Claims** for the contracting states: BE CH DE FR GB IT LI LU NL SE

1) Derivatives of 1,3-dihydro-4-(1-hydroxy-2-aminoethyl)-2H-indol-2-one, as well as their salts of addition with mineral or organic acids, characterized in that they answer to the general formula I

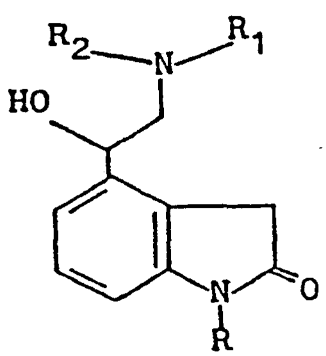

(I)

in which R represents a hydrogen atom or an alkyl radical containing from 1 to 6 carbon atoms or a phenyl alkyl radical containing from 7 to 10 carbon atoms, $R_1$ and $R_2$ represent a hydrogen atom, a linear or branched alkyl radical containing from 1 to 8 carbon atoms, possibly substituted, except in the case of the methyl radical, by one or more halogen atoms or by one or more hydroxy, phenyl, or phenoxy radicals, or phenyl or phenoxy radicals substituted by one or more halogens, hydroxy, methyl or methoxy radicals, or $R_1$ and $R_2$ represent a cycloalkyl radical containing from 3 to 7 carbon atoms, a cycloalkylalkyl radical containing from 4 to 7 carbon atoms or an allyl or propargyl radical.

2) Derivatives of 1,3-dihydro-4-(1-hydroxy-2-aminoethyl)-2H-indol-2-one as defined by formula I of claim 1, as well as their salts of addition with mineral or organic acids, characterized in that R represents a hydrogen atom

or an alkyl radical containing from 1 to 3 carbon atoms, $R_1$ and $R_2$ represent a hydrogen atom or a possibly substituted linear or branched alkyl radical containing from 1 to 8 carbon atoms.

3) Derivatives of 1,3-dihydro-4-(1-hydroxy-2-aminoethyl)-2H-indol-2-one, according to claim 2, as well as their salts of addition with mineral or organic acids, characterized in that R represents a hydrogen atom, and $R_1$ and $R_2$ represent a hydrogen atom or a linear or branched alkyl radical containing from 1 to 8 carbon atoms, possibly substituted by a phenyl or phenoxy radical, these latter radicals themselves being possibly substituted by a halogen atom.

4) 1,3-dihydro-4-[1-hydroxy-2-(1-methyl-ethylamino)ethyl -2H-indol-2-one, as well as its salts of addition with mineral or organic acids.

5) Preparation process of derivatives answering to formula I of claim 1, as well as their salts of addition with mineral or organic acids, characterized in that a product with the formula II:

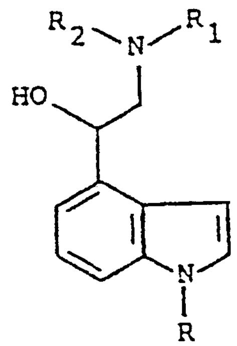

(II)

in which R, $R_1$ and $R_2$ have the significance already indicated in claim 1, is submitted to the action of a halogenation agent, so as to obtain a product with the formula III:

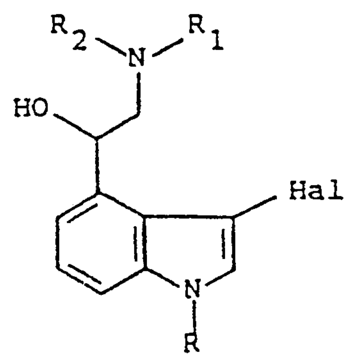

(III)

in which Hal represents a halogen atom and R, $R_1$ and $R_2$ have the significance already indicated, which is hydrolized so as to obtain a product with the formula I:

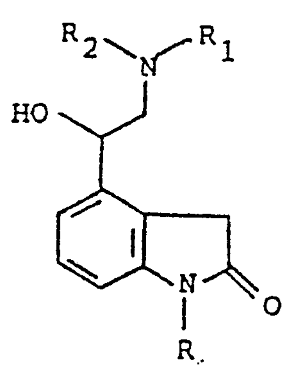

(I)

in which R, $R_1$ and $R_2$ have the significance already indicated, which is isolated and if desired, salified.

6) Process according to claim 5, characterized in that

- the halogenation agent is an N-halosuccinimide,
- hydrolysis is carried out by means of a mineral acid in an aqueous solution.

7) Medicaments characterized in that they are composed of new derivatives of 1,3-dihydro-4-(1-hydroxy-2-aminoethyl)-2H-indol-2-one, as defined by formula I of claim 1, as well as by their salts of addition with pharmaceutically acceptable acids.

8) Medicaments, characterized in that they are composed of new derivatives of 1,3-dihydro-4-(1-hydroxy-2-aminoethyl)-2H-indol-2-one, as defined in claim 2 or 3, as well as by their salts of addition with pharmaceutically acceptable acids.

9) Medicaments, characterized in that they are composed of the derivative of 1,3-dihydro-4-(1-hydroxy-2-aminoethyl)-2H-indol-2-one, as defined in claim 4, as well as by its salts of addition with pharmaceutically acceptable acids.

10) Pharmaceutical compositions, characterized in that they contain, as active principle, at least one of the medicaments as defined in one of the claims 7, 8 or 9.

**Claims** for the contracting state: AT

1) Process for preparing new derivatives of 1,3-dihydro-4-(1-hydroxy-2-aminoethyl)-2H-indol-2-one, as well as their salts of addition with mineral or organic acids answering to the general formula (I)

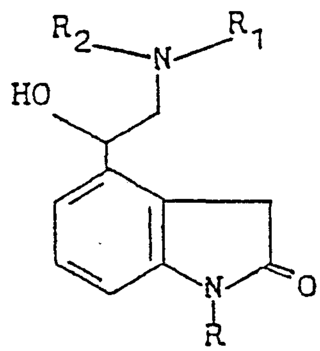

(I)

in which R represents a hydrogen atom or an alkyl radical containing from 1 to 6 carbon atoms or a phenyl alkyl containing from 7 to 10 carbon atoms, $R_1$ and $R_2$ represent a hydrogen atom, a linear or branched alkyl radical containing from 1 to 8 carbon atoms, possibly substituted, except in the case of the methyl radical, by one or more halogen atoms or by one or more hydroxy, phenyl, or phenoxy radicals, or phenyl or phenoxy radicals substituted by one or more halogens, hydroxy, methyl or methoxy radicals, or $R_1$ and $R_2$ represent a cycloalkyl radical containing from 3 to 7 carbon atoms, a cycloalkylalkyl radical containing from 4 to 7 carbon atoms or an allyl or propargyl radical, characterized in that a product with the formula (II):

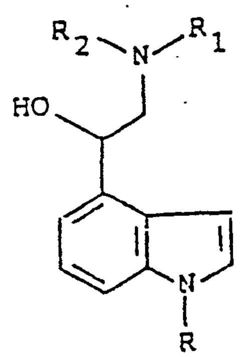

(II)

in which R, $R_1$ and $R_2$ have the significance already indicated, is submitted to the action of a halogenation agent, so as to obtain a product with the formula III:

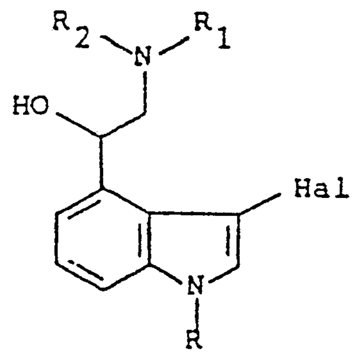

(III)

in which Hal represents a halogen atom and R, $R_1$ and $R_2$ have the significance already indicated, which is hydrolized so as to obtain a product with the formula I:

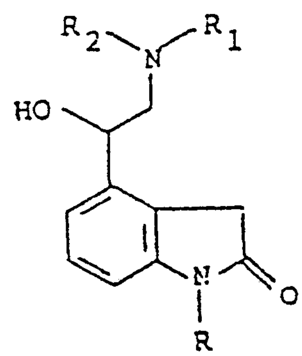

(I)

in which R, $R_1$ and $R_2$ have the significance already indicated, which is isolated and if desired, salified.

2) Process according to claim 1, characterized in that

- the halogenation agent is an N-halosuccinimide,
- hydrolysis is carried out by means of a mineral acid in an aqueous solution.

3) Process according to claim 1 or 2, characterized in that at the start a product with the formula II is used in which R represents a hydrogen atom or an alkyl radical containing from 1 to 3 carbon atoms, $R_1$ and $R_2$ represent a hydrogen atom or a possibly substituted linear or branched alkyl radical containing from 1 to 8 carbon atoms.

4) Process according to claim 1 or 2, characterized in that at the start a product with the formula II is used in which R represents a hydrogen atom, and $R_1$ and $R_2$ represent a hydrogen atom or a linear or branched alkyl radical containing from 1 to 8 carbon atoms, possibly substituted by a phenyl or phenoxy radical, these latter radicals themselves being possibly substituted by a halogen atom.

5) Process according to claim 1 or 2, characterized in that 1,3-dihydro-4-[1-hydroxy-2-(1-methylethylamino)-ethyl]-2H-indol-2-one is prepared as well as its salts of addition with mineral or organic acids.